# EUROPEAN PATENT SPECIFICATION

(11) **EP 3 514 233 B1**
(45) Date of publication and mention of the grant of the patent: **02.03.2022**
(21) Application number: 17850912.1
(22) Date of filing: 13.09.2017
(51) Int. Cl.: C12N 15/09, C12Q 1/6806, C07H 1/08

(54) **METHOD FOR RECOVERING CELL-FREE DNA**
VERFAHREN ZUR RÜCKGEWINNUNG VON ZELLFREIER DNA
PROCÉDÉ DE RÉCUPÉRATION D'ADN ACELLULAIRE

(30) Priority: 14.09.2016 JP 2016179451
(43) Date of publication of application: 24.07.2019
(73) Proprietor: Toray Industries, Inc., Tokyo 103-8666 (JP)
(72) Inventor: NAKAGAWA, Mai, Kamakura-shi Kanagawa 248-8555 (JP); SEKIGUCHI, Shota, Kamakura-shi Kanagawa 248-8555 (JP); SUDO, Hiroko, Kamakura-shi Kanagawa 248-8555 (JP)
(74) Representative: Kador & Partner PartG mbB
(86) International application number: PCT/JP2017/033011
(87) International publication number: WO 2018/052011

(56) References cited:
- EP-A1- 2 602 321
- EP-A1- 3 272 866
- WO-A1-2015/132615
- DE-A1- 4 321 904
- JP-A- 2003 235 555
- JP-A- 2003 235 555
- JP-A- 2007 006 728
- JP-A- 2007 529 229
- US-A1- 2006 166 241
- M. ARAMESH ET AL: "Ultra-high-density 3D DNA arrays within nanoporous biocompatible membranes for single-molecule-level detection and purification of circulating nucleic acids", NANOSCALE, vol. 7, no. 14, 26 February 2015 (2015-02-26), pages 5998-6006, XP055394309, United Kingdom ISSN: 2040-3364, DOI: 10.1039/C4NR07351G

## Description

### [TECHNICAL FIELD]

The present invention relates to a method of collecting cell-free DNA.

### [BACKGROUND ART]

The development of experimental techniques using nucleic acids has allowed for a search for a new gene and an analysis of the gene. Screening tests and clinical tests by using gene analysis have been performed in the medical field as well, for example, a human genome has been analyzed in order to identify a disease such as a cancer, and a pathogen genome has been analyzed in order to identify a pathogen infection.

Recently, cell-free DNA among nucleic acids is especially attracting interest, clinically oriented researches, such as a research for determining the presence or absence of an effect on a therapeutic agent by analyzing a cancer-specific genetic mutation which mutation is present in cell-free DNA from a tumor, have been actively carried out.

For example, therapeutic agents, such as erlotinib for a patient suffering from lung cancer in which EGFR genetic mutation was detected, vemurafenib for a patient suffering from skin cancer in which BRAF genetic mutation was detected and rucaparib for a patient suffering from ovarian cancer in which BRCA genetic mutation was detected, have been approved. It is also known that the effect of therapeutic agents targeted to these genetic mutations are common throughout the types of cancers, for example, darafenib, a therapeutic agent for V600 mutation of BRAF gene, is recommended to both of a skin cancer patient and a non-small-cell lung cancer patient.

However, so far, a gene is generally collected from a surgically resected cancer tissue to obtain a cancer-specific gene. Obtaining a resected tissue is highly invasive to a patient, and is problematic in that the result varies depending on which site of various cancer tissues is taken, and that it is not suitable to pre-operative treatment, and the like. Recently, it is reported that a cancer-specific genetic mutation may be present in cell-free DNA present in body fluid, it is desired that a genetic mutation is therefore detected without resecting a cancer tissue.

A larger number of cell-free DNAs in blood of a patient suffering from a cancer are contained compared to one without cancer due to releasing of cell-free DNAs from cancer cells to blood when suffering from a cancer (Patent Document 7). Therefore, if the amount of cell-free DNA can be accurately measured, then it is possible to detect a cancer low-invasively and conveniently by using a body fluid sample. Patent Document 7 describes the method of detecting a cancer based on the amount of cell-free DNA collected by a silica gel membrane.

However, the amount of cell-free DNA present in a body fluid is small, in addition to which it is required that to detect cell-free DNA which is referred to as cell-free tumor DNA (ctDNA) and which is originated from a tumor cell which has an allele frequency of only 0.1% to a few percent in total DNAs in blood among cell-free DNAs in order to analyze a cancer-specific mutation site which is present in cell-free DNA sequence, and a technology for collecting cell-free DNA in a high yield is required.

Exemplary examples of methods of collecting nucleic acids which are generally performed currently include, as representative methods, phenol-chloroform extraction, ethanol precipitation and nucleic acid adsorption on silica. Among these, the most common method is the Boom method, as described in Patent Document 1, in which nucleic acids are adsorbed on a metal oxide containing silica, then eluted, and collected. This method is characterized by the concentration of the nucleic acids along with the collection of the nucleic acids from the nucleic acid-adsorbed silica by a centrifugation operation.

Patent Document 2 describes a technique of adsorbing cell-free DNA on silica based on the Boom method. According to this, when phenol-chloroform extraction is performed under acidic conditions, RNA and DNA are separated into an aqueous layer and a chloroform layer, respectively. Under the neutral conditions, RNA and DNA are partitioned into an aqueous phase. That is, it describes that a nucleic acid to be acquired can be selectively extracted by changing the condition of an extraction solution.

However, the methods described in Patent Documents 1 and 2 requires the use of organic solvents such as alcohols in the step of adsorbing the nucleic acids, which causes a problem of disposal of the solvents and also a more complicated collection operation. In addition, there is also a problem in which the isolated nucleic acids are contaminated with these organic solvents, and the later detection reaction is affected.

Patent Document 3 describes that a nucleic acid having the length of not less than 300 base pairs and not more than 1000 base pairs exhibits inferior adsorption property on silica compared to the adsorption property of a shorter nucleic acid. Thus, it is assumed that, in collecting even shorter cell-free DNA, methods using silica is not suitable.

Patent Documents 4 and 5 do not describe about any cell-free DNAs, but they describe methods of collecting nucleic acids without using any organic solvents. Patent Document 4 describes a method of adsorbing nucleic acids on alpha aluminum oxide particles, zirconia particles, titania particles, or the like to collect the nucleic acids efficiently. Patent Document 5 describes a method of adsorbing and collecting nucleic acids by means of the principle of ion exchange chromatography and suggests that aluminum oxide can be used as an anion-exchange material.

Patent Document 6 describes that nucleic acids can be allowed to bond strongly to alpha aluminum oxide and gamma aluminum oxide or, in an opposite manner, prevented the bond, depending on a solution in which the nucleic acids are dissolved. In addition, it also describes that the bonded nucleic acids are hardly eluted even after repeating washing.

Patent Document 8 discloses a method for isolating a single-stranded nucleic acid and/or a double-stranded nucleic acid from a sample containing the single-stranded nucleic acid and/or the double-stranded nucleic acid.

Patent Document 9 is directed to The present invention relates to a method for selectively amplifying a target nucleic acid from a biological sample containing a mixture of the target nucleic acid and non-target nucleic acid.

Patent Document 10 relates to a method of isolating and purifying nucleic acids using an intercalator.

Patent Document 11 discloses a method for chromatographic purification and separation of mixtures nucleic acids.

Patent Document 12 relates to a method for collecting nucleic acids, an aluminum oxide support with a water-soluble neutral polymer adsorbed on the surface thereof, and a kit for collecting nucleic acids.

Non-Patent Document 1 relates to a new broad-range sensor platform for ultrasensitive and selective detection of circulating DNA down to the single-molecule level.

### [PRIOR ART REFERENCES]

### [PATENT DOCUMENTS]

[Patent Document 1] US5,234,809 B
[Patent Document 2] WO2016/007755
[Patent Document 3] Japanese National-Phase Publication No. 2011-522529
[Patent Document 4] WO92/18514
[Patent Document 5] Japanese National-Phase Publication No. 2013-505719
[Patent Document 6] Japanese National-Phase Publication No. 2005-505269
[Patent Document 7] WO2015/114641
[Patent Document 8] Japanese National-Phase Publication No. 2003-235555
[Patent Document 9] EP 2 602 321 A1
[Patent Document 10] US 2006/166241 A1
[Patent Document 11] DE 43 21 904 A1
[Patent Document 12] EP 3 272 866 A1

### [NON-PATENT DOCUMENTS]

[Non-Patent Document 1] M. ARAMESH ET AL: "Ultra-high-density 3D DNA arrays within nanoporous biocompatible membranes for single-molecule-level detection and purification of circulating nucleic acids", NANOSCALE, vol. 7, no. 14, 26 February 2015 (2015-02-26), pages 5998-6006, XP055394309, United Kingdom ISSN: 2040-3364, DOI: 10.1039/C4NR07351G

### [SUMMARY OF THE INVENTION]

### [PROBLEMS TO BE SOLVED BY THE INVENTION]

The present inventors investigated a method of collecting cell-free DNA by using aluminum oxide in order to collect cell-free DNA in a high yield. For investigating the collecting method, the present inventors investigated whether cell-free DNA could be collected by the methods of collecting nucleic acids using aluminum oxides described in Patent Document 4 and 5 above. However, as shown in Comparative Example 1 described below, these methods could not allow any cell-free DNAs to be collected.

An object of the present invention is that a method of collecting a cell-free DNA by using aluminum oxide in order to collect cell-free DNA in a high yield is established.

### [MEANS FOR SOLVING THE PROBLEMS]

The present inventors have discovered that adsorbing a water-soluble neutral polymer on the surface of aluminum oxide allows cell-free DNA to be efficiently collected.

The present invention includes the following (1) to (8).
(1) A method of collecting cell-free DNA from a body fluid sample, comprising the following steps of:
   step a) of mixing an aluminum oxide support comprising a water-soluble neutral polymer adsorbed on a surface thereof, wherein the support is produced by adsorbing a water-soluble neutral polymer onto the surface of aluminum oxide, with the body fluid sample to adsorb the cell-free DNA onto the support, wherein the polymer is polyethylene glycol, polyvinyl alcohol, polyvinylpyrrolidone, poly(2-ethyl-2-oxazoline) or hydroxypropylmethylcellulose,
   step b) separating the support on which the cell-free DNA was adsorbed from the mixture mixed in the step a), and
   step c) collecting the cell-free DNA by adding an eluent to the support on which the cell-free DNA was adsorbed and which was separated in the step b).
(2) The method of collecting cell-free DNA according to (1), in which the body fluid sample is whole blood, blood serum, blood plasma, urine, or saliva.
(3) The method of collecting cell-free DNA according to (1) or (2), in which the water-soluble neutral polymer is a polymer having a zeta potential of not less than -10 mV and not more than +10 mV in a solution of pH 7.
(4) The method of collecting cell-free DNA according to any of (1) to (3), in which the eluent is a buffer solution.
(5) The method of collecting cell-free DNA according to any of (1) to (4), in which the body fluid sample is treated with a protein denaturant.
(6) The method of collecting cell-free DNA according to (5), in which the protein denaturant is a chaotropic salt or protein-denaturing enzyme.
(7) A method of detecting a genetic mutation specific to cancer by analyzing a genetic sequence of cell-free DNA, in which the method comprises the steps of collecting the cell-free DNA from a body fluid sample of a subject using the method of collecting cell-free DNA according to any of (1) to (6), and detecting the genetic mutation specific to cancer from the cell-free DNA thus collected.
(8) A method of detecting a cancer by comparing an amount of cell-free DNA from a subject with an amount of cell-free DNA from a specimen without cancer, in which the method comprises the steps of collecting the cell-free DNA from the respective body fluid sample from the subject and the specimen without cancer using the method of collecting cell-free DNA according to any of (1) to (6), and detecting the cancer by comparing the amount of cell-free DNA from a subject with the amount of a cell-free DNA from a specimen without cancer.

### [EFFECT OF THE INVENTION]

The method of collecting cell-free DNA of the present invention allows for collecting cell-free DNA in a high yield from a small amount of a sample using a simple method. In addition, according to the collecting method of the present invention, the detectability for a genetic mutation specific to cancer and for a cancer is improved because cell-free DNA can be collected in a high yield.

### [BRIEF DESCRIPTION OF DRAWINGS]

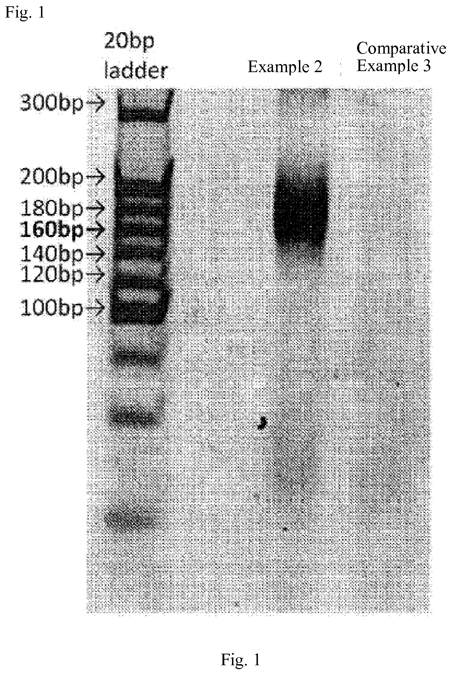

Figure 1 shows an electropherogram of cell-free DNAs collected by using the method of collecting cell-free DNA of the present invention and cell-free DNA collected by using a commercially available kit of collecting cell-free DNA.

### [MODE FOR CARRYING OUT THE INVENTION]

Cell-free DNA to be collected by the method of collecting cell-free DNA of the present invention is a generic term for cell-free normal DNA originated from a normal cell which is leaked into a body fluid and cell-free tumor DNA (ctDNA) originated from a tumor cell, is herein described as cell-free DNA.

A length of cell-free DNA is about 166 bp which is generally equivalent to one unit of histone, while cell-free DNA having a characterized length may be detected in a patient suffering from cancer and/or infested with a parasite. For example, in a specimen from a patient suffering from a cancer, cell-free DNAs with lengths of base pairs of 332 bp (166 bp × 2), 498 bp (166 bp × 3), 664 bp (166 bp × 4) and the like are contained because the specimen is not digested to such an extent as to be broken down into one unit of histone. It is known that cell-free DNAs having lengths of base pairs of 205 bp and the like are leaked into blood serum in case of a patient infested with a plasmodium falciparum, and cell-free DNAs having lengths of base pairs of 70 bp and the like are leaked into urine in case of a patient infested with a leishmania (Trends in Parasitology, May 2016,Vo.32,No.5).

The length of cell-free DNA collected in the present invention is not particularly limited, but cell-free DNA having a length of not more than 500 bp can be efficiently collected.

A body fluid sample used in the present invention is not particularly limited as long as it is any body fluid sample containing cell-free DNA, but for example, whole blood, blood plasma, blood serum, urine and saliva, and preferably blood plasma or blood serum can be used.

Body fluid samples which are not treated can be used, but those which is treated with a protein denaturant can be also used in order to collect cell-free DNA in higher yield.

A protein denaturant used in the present invention is not particularly limited, but, for example, a surfactant such as an SDS, sarcosyl and CTAB; a protein-denaturing enzyme such as a Proteinase K which is a type of serine proteases and broadly specific to cleavage; a chaotropic salt such as guanidinium chloride, guanidine thiocyanate salt and urea; and other mercaptoethanols can be preferably used. Also, a commercially available buffer solution containing a protein denaturant can be preferably used and, for example, Buffer RLT (manufactured by QIAGEN K.K.) which contains guanidine thiocyanate salt, etc. can be preferably used as a protein denaturant for a chaotropic salt. Among these protein denaturants, a chaotropic salt or protein-denaturing enzyme is especially preferred.

A protein denaturant may be used alone or in combination of plural types. The examples of treatments in combination of protein denaturants include as follows. For example, after addition of 1% SDS, Proteinase K can be added to a body fluid sample, and the resulting mixture can be then heated at 60°C for 20 minutes. Not less than 4 M guanidinium chloride, guanidine thiocyanate salt or urea can be added to a body fluid sample, followed by addition of sarcosyl so that the final concentration is 0.5% or more, or to addition of mercaptoethanol so that the final concentration is 50 mM or more.

In the above-mentioned operations, an inhibitor of a degradative enzyme for a nucleic acid may be added to suppress the degradation of cell-free DNA contained in the body fluid sample. The inhibitor of the enzyme, for example, EDTA can be added in a final concentration of 1 mM or less to the body fluid sample. As an inhibitor of ribonuclease, commercially available RNasin (registered trademark) Plus Ribonuclease Inhibitor (Promega Corporation), Ribonuclease Inhibitor (TAKARA BIO INC.), RNase inhibitor (TOYOBO CO., LTD.), and the like can be used.

In the present invention, a body fluid sample is optionally diluted. The solution to be used for dilution is not particularly limited, but a solution which is widely used with a solution containing a nucleic acid, such as water or a Tris-hydrochloride buffer solution is preferably used. A solution containing the above protein denaturant may be used. When the body fluid sample is diluted with the solution containing the protein denaturant, for example, 4 M or more guanidinium chloride, guanidine thiocyanate salt, or urea can be used for dilution.

The present invention achieves the high-yield collection of a cell-free DNA by using an aluminum oxide support comprising a water-soluble neutral polymer adsorbed on the surface thereof. In the present invention, the aluminum oxide support comprising a water-soluble neutral polymer adsorbed on the surface thereof is referred to a support comprising the water-soluble neutral polymer adsorbed around granular aluminum oxide. Thereinafter, the support is described as the support of the present invention.

In the present invention, adsorption of cell-free DNA onto a support is referred to adsorption in which an adsorbate can be released reversibly.

Examples of methods of quantifying the amount of a nucleic acid in quantifying cell-free DNA include a spectrophotometry, a fluorescence measurement, a luminescence measurement, electrophoresis, PCR, digital PCR, real-time PCR, an analysis using a microarray, and an analysis using a sequencer. Specifically, in a quantitative method of a spectrophotometry, in case of an unmodified nucleic acid, the amount of the nucleic acid can be quantified by measuring the absorbance at 260 nm. In a quantitative method of a fluorescence measurement, the amount of a nucleic acid can be quantified by modifying the nucleic acid with a fluorescent dye, and comparing the fluorescence intensity derived from the fluorescent dye with the fluorescence intensity of a solution of a known concentration. In a quantitative method of an electrophoresis, the amount of the nucleic acid can be determined by migrating a sample collected simultaneously with a sample of a known concentration and then comparing a dye concentration of a band by image-analyzing after staining a gel.

PCR is an abbreviation of polymerase chain reaction, can allow a specific sequence among a DNA sample to be selectively amplified. In PCR, the initial concentration of a nucleic acid can be determined with the amount of the product after the end of amplification reaction of the nucleic acid.

Real-time PCR is referred to as a quantitative real-time PCR, allows for relative quantification of a DNA which serves as a template based on an amplification efficiency by continuously measuring the amplification by PCR. In addition, when a calibration curve of amplification efficiency is made by using a standard sample, the absolute amount can be also quantified. The amplification efficiency is calculated as a number of amplification cycles (Cq value), smaller value indicates more amount of a nucleic acid.

In a digital PCR, a PCR reaction is carried out after dispensing DNA samples into fine partitions and the amount of a nucleic acid can be quantified from the signal strength of each fraction after the PCR reaction.

In real-time PCR, the length of a collected cell-free DNA can be detected by designing a primer suitably. For example, the length of cell-free DNA can be detected by amplification of any sequence of beta-actin known as a housekeeping gene. For example, for confirming that the cell-free DNA of 166 bp is collected, a primer which can amplify DNA of about 100 bp among beta-actin genes can be used (W.SUN et al., The role of plasma cell-free DNA detection in predicting operative chemoradiotherapy response in rectal cancer patients. ONCOLOGY REPORTS 31:1466-1472,2014). Specifically, the collected cell-free DNA can be detected using the primers of SEQ NO:1 and SEQ NO:2 which can amplify the DNA having the length of 93 bp among beta-actin genes.

In real-time PCR, generally, when the number of amplification cycles (Cq value) is 40 or more, it is below the detectable limit.

A polymer used in the present invention is a generic term for compounds in which a large number of repeating units, called a monomer which is a basic unit, are connected. The above-mentioned polymers include any of homopolymers consisting of one kind of monomer and copolymers composed of two or more kinds of monomers, and polymers with an arbitrary degree of polymerization can be used. The above-mentioned polymers include any of naturally-occurring polymers and synthetic polymers.

The water-soluble neutral polymer used in the present invention is a polymer which has water-soluble property and the solubility in water of at least 0.0001 wt% or more, preferably 0.001 wt% or more, more preferably 0.01 wt% or more, and further preferably 0.1 wt% or more.

The water-soluble neutral polymer used in the present invention is preferably a polymer with a zeta potential of not less than -10 mV and not more than +10 mV in a solution of pH 7. More preferably, the water-soluble neutral polymer used in the present invention is a polymer with a zeta potential of not less than -8 mV and not more than +8 mV, further preferably not less than -6 mV and not more than +6 mV, and particularly preferably not less than -4.0 mV and not more than +1.1 mV.

The zeta potential is one of values indicating electrical properties on colloidal interfaces in a solution. When charged colloids are dispersed in a solution, on the surface of a colloid, an electrical double layer is formed by counter ions with respect to the charge of the colloidal surface. The electrical potential on this colloidal surface is called surface potential. Because the electrical double layer is formed by electrostatic interaction between the colloidal surface charges, ions are more strongly fixed as they are closer toward the colloid. In the electrical double layer, a layer in which counter ions are strongly fixed on the colloidal surface by electrostatic interaction is called a stern layer, and the potential of the stern layer is called a stern potential. When a colloid is moved relative to a solution, the stern layer is also moved together with the colloid. In this case, there is a boundary surface that is moved together with the colloid outside the stern layer as viewed from the colloid due to the viscosity of the solution. This surface is called a slipping plane. The potential of this slipping plane is defined as a zeta potential when the potential at a point sufficiently far from the colloid is defined as zero. Thus, as the zeta potential varies depending on the colloidal surface charge and the surface charge changes according to protonation and deprotonation which depend on pH, the value in a solution of pH 7 is used as a standard in the present invention. Because the distance from the colloidal surface to the slipping plane is generally small compared to the colloidal size, the colloidal surface can be represented approximately as the slipping plane. In case of the water-soluble neutral polymer used in the present invention as well, the colloidal surface potential dispersed in the solution can be considered as the zeta potential.

In the present invention, a zeta potential is measured using laser doppler electrophoresis on ELS-Z from Otsuka Electronics Co., Ltd. The laser Doppler electrophoresis method is a measurement method which utilizes the Doppler effect which is the change in the frequency of light or sound waves when the light or sound waves strike an object in motion due to electrophoresis, and scatter or reflect.

When the zeta potential of a polymer is measured, a polymer solution can be prepared as a colloidal dispersion to measure its zeta potential. For example, a polymer is dissolved in an electrolyte such as a phosphate buffer solution, a sodium chloride solution, and a citrate buffer solution to prepare a polymer solution, which is then measured by detecting scattered light and reflected light of the polymer dispersed in the solution. A bigger colloid size allows for the detection of scattered light and reflected light under a lower concentration.

Specific conditions for measuring the zeta potential of a polymer by the laser Doppler method is not particularly limited, but the zeta potential of the polymer can be measured as follows, for example: the polymer is dissolved in a phosphate buffer solution (10 mM, pH 7) under the concentration of not less than 1 wt% and not more than 10 wt%; this solution is then placed in a cell for measurement and installed in a zeta potential measurement instrument which utilizes the principle of the laser Doppler electrophoresis method, and thus the zeta potential can be measured at room temperature.

The water-soluble neutral polymers used in the present invention are polyvinyl alcohol, polyvinylpyrrolidone, polyethylene glycol, poly(2-ethyl-2-oxazoline) or (hydroxypropyl)methylcellulose.

In addition, polysaccharides or polysaccharide analogues such as ficoll, agarose, chitin and dextran as well as proteins and peptides such as albumin are also included within the water-soluble neutral polymer in the present invention.

A portion of functional groups of the water-soluble neutral polymer may be ionized or substituted with a functional group exhibiting positive or negative charge, or a functional group exhibiting solubility in water such as an acetyl group may be introduced to side chains.

In the present invention, the molecular weight of the water-soluble neutral polymer is, for example, preferably 0.4 kD or more, and more preferably 6 kD or more.

The aluminum oxide used in the present invention is an amphoteric oxide of the composition formula Al₂O₃, also referred to as alumina.

For the aluminum oxide, naturally-occurring aluminum oxide or aluminum oxide manufactured industrially may be used. Examples of methods for producing aluminum oxide include the Bayer method in which gibbsite is used as a starting material, an alkoxide method, a method via a hydroxide in the form of boehmite (also called sol-gel method) such as a neutralization method or an oil droplet method, an aluminum salt thermal decomposition method, or an anodic oxidation method.

Aluminum oxide manufactured industrially can be available from reagent manufacturers, catalyst chemical manufacturers, the Committee of Reference Catalyst of the Catalysis Society of Japan, and the like.

Depending on the crystal structure, aluminum oxide is classified as alpha aluminum oxide, rho aluminum oxide, khi aluminum oxide, kappa aluminum oxide, eta aluminum oxide, gamma aluminum oxide, delta aluminum oxide, theta aluminum oxide, or the like. In the present invention, gamma aluminum oxide with a high specific surface area is preferred.

Furthermore aluminum oxide changes its acidic sites (Al⁺, Al-OH₂⁺) and basic sites (Al-O⁻) depending on the calcination temperature during the production. Depending on the number of acidic sites and basic sites of the aluminum oxide, the aluminum oxide works as acidic alumina if there are more acidic sites, as basic alumina if there are more basic sites, and as neutral alumina if the acidic and basic sites each is almost in the same quantity. The difference in these properties can be confirmed by the addition of a pH indicator, BTB solution. It is confirmed that, when a BTB solution is added, if aluminum oxide turns yellow, then the aluminum oxide is acidic alumina; if aluminum oxide turns green, then it is neutral alumina; and if the aluminum oxide turns blue, then it is basic alumina. In the present invention, any aluminum oxide can be used regardless of such a difference in property.

Aluminum oxide used in the present invention is preferably in a granular form. The particle size may be uniform, or those with different particle sizes may be combined in use. For example, the aluminum oxide having a particle size of less than 212 µm can be preferably used, more preferably the aluminum oxide having a particle size of less than 100 µm can be used.

In the present invention, the particle size is defined by an aperture size of a sieve based on JIS Z-8801-1: 2006 according to Japanese Industrial Standards. For example, in the aperture size according to the above JIS standard, particles which can pass through the sieve of 40 µm and cannot pass through the sieve of 32 µm will have the particle size of not less than 32 µm and less than 40 µm.

The eluent used in the present invention is not particularly limited as long as cell-free DNA adsorbed on the support of the present invention can be eluted, but is preferably a buffer solution, and the buffer solution may contain a chelating agent. Specific examples thereof include a citrate buffer solution containing citric acid and sodium citrate, a phosphate buffer solution containing phosphoric acid and sodium phosphate, and a Tris-EDTA buffer solution obtained by adding EDTA to a Tris-hydrochloric acid buffer solution containing tris(hydroxymethyl)aminomethane and hydrochloric acid.

The pH of the buffer solution is preferably pH 4 or more and pH 9 or less, more preferably pH 5 or more and pH 8 or less.

The buffer solution used in the present invention can be prepared as follows. For example, a 0.5 M phosphate buffer solution (pH 7) is prepared as follows. A 0.5M aqueous solution of disodium hydrogen phosphate and 0.5M sodium dihydrogen phosphate are prepared. While measuring the pH, a sodium dihydrogen phosphate solution is added to the 0.5 M aqueous solution of disodium hydrogen phosphate until the pH reaches pH7. In a similar way, a buffer solution of another pH can be also prepared.

As a chelating agent contained in the buffer solution, a substance that has a ligand with a plurality of coordination positions, and binds to a metal ion to form a complex can be used.

Specific examples of chelating agents include ethylenediaminetetraacetic acid (EDTA), nitrilotriacetic acid (NTA), glycol ether diaminetetraacetic acid (EGTA), polyphosphoric acid, metaphosphoric acid and/or salts thereof. The final concentration of the chelating agent is not particularly limited as long as it is 50 mM or more, and is preferably 100 mM or more, and further preferably 500 mM or more.

Examples of compounds as a chelating agent other than the above include anionic polymers. Since a polymer including a carboxylic acid group on its side chain coordinates to a metal ion, the buffer solution may contain such a polymer. Examples of polymers having such a function include polyvinyl sulfonic acid and/or salt thereof. The final concentration is not particularly limited as long as it is 1 wt% or more, and preferably 10 wt% or more.

The present invention is a method for collecting a cell-free DNA from a body fluid sample, wherein the method comprises step a) of mixing an aluminum oxide support comprising a water-soluble neutral polymer adsorbed on the surface thereof, wherein the support is produced by adsorbing a water-soluble neutral polymer onto the surface of aluminum oxide, and the body fluid sample to adsorb the cell-free DNA onto the support, wherein the polymer is polyethylene glycol, polyvinyl alcohol, polyvinylpyrrolidone, poly(2-ethyl-2-oxazoline) or hydroxypropylmethylcellulose, step b) of separating the support on which the cell-free DNA was adsorbed from the mixture mixed in the step a), and step c) of adding an eluent to the support on which the cell-free DNA was adsorbed in the step b) to collect the cell-free DNA. Each step will be described in detail below.

The support used in the present invention is produced by adsorbing the water-soluble neutral polymer onto the surface of aluminum oxide. The surface coverage ratio with polymer is not less than 7%, and preferably not less than 40%. The water-soluble neutral polymer may not be necessarily adsorbed in an even thickness.

In the present invention, the coverage ratio of aluminum oxide with polymer is calculated by analyzing a potential distribution map obtained by a surface potential microscope (also known as Kelvin probe force microscope; KFM). As a surface potential microscope, for example, NanoScope Iva AFM Dimension 3100 stage AFM system, manufactured by Digital Instruments, from Bruker AXS can be used.

When the surface coverage ratio is calculated by means of the surface potential microscope, the scale of the field of view for measurement is within a range of 0.5 µm × 1 µm. The surface coverage ratio is calculated as follows. First, the surface potential image of the aluminum oxide is obtained to calculate the average potential in the field of view. Subsequently, the surface potential image of the water-soluble neutral polymer is obtained to calculate the average potential in the field of view. The surface potential image of the aluminum oxide on which the water-soluble neutral polymer is adsorbed is then obtained to calculate the average potential in the field of view. The coverage ratio of the aluminum oxide alone is considered as 0% and that of the water-soluble neutral polymer alone is considered as 100%. The ratio of the average potential of the aluminum oxide on which the water-soluble neutral polymer is adsorbed to that of the water-soluble neutral polymer is obtained, and thus the surface coverage ratio of the aluminum oxide on which the water-soluble neutral polymer is adsorbed is calculated. When the surface coverage ratio is calculated, for the average potential in each field of view to be used, three free particles of the present invention are selected randomly, and each average value of measured values is used.

Photoshop from Adobe Systems Incorporated or the like can be also used as an image analysis software for calculating the surface coverage ratio. In this instance, for the image analysis, the average value of the surface potential of the aluminum oxide is set as a lower limit of the scale, the average value of the surface potential of the water-soluble neutral polymer is set as an upper limit of the scale, the lower limit color is set as black (8 bits, RGB value 0), and the upper limit color is set to red (R value 255), green (G value 255), or blue (B value 255), or the like. The surface potential image of the aluminum oxide on which the water-soluble neutral polymer is adsorbed is displayed in the scale set as above, and either the R, G, or B value is divided by 255, and the ratio is decided as the surface coverage ratio.

Before the water-soluble neutral polymer is adsorbed on the surface, the aluminum oxide may be washed in advance with a solution such as water or ethanol to remove the impurities adsorbed on the surface, or this washing step may be omitted.

Examples of methods of adsorbing the water-soluble neutral polymer on aluminum oxide include a method in which the water-soluble neutral polymer is dissolved to prepare a polymer solution and brought the solution into contact with the aluminum oxide. Specifically, aluminum oxide may be dipped into the polymer solution, the polymer solution may be added dropwise to aluminum oxide, the polymer solution may be coated on aluminum oxide, or the polymer solution may be sprayed in the form of a mist onto the aluminum oxide.

The methods for dipping aluminum oxide into the polymer solution are not particularly limited. For example, it may be stirred by pipetting or mixing by inversion, or by a disperser such as a stirrer, mixer, vortex or mill, or an ultrasonic processor.

The concentration of the water-soluble neutral polymer is not particularly limited, but it is preferably 0.01 wt% or more, more preferably, 0.1 wt% or more.

The mixing time for stirring is not particularly limited as long as the polymer and the aluminum oxide are mixed evenly, but in the case of a vortex, the mixture is stirred for 1 minute or more, and preferably 5 minutes or more.

The aluminum oxide can also be dip-coated into the polymer solution using a sifter or a sieve. The mixing time for dipping in the solution may be, in the case of the polymer concentration of 0.1 wt% or more, 5 minutes or more, and preferably 30 minutes or more.

When the polymer solution is added dropwise, a dropper, a dropping funnel or the like can be used. When the polymer solution is added dropwise, the aluminum oxide may also be shaken or rotated, or a spin coater or the like may be used.

When the polymer solution is coated, a brush, roller or a wire bar can be used.

When the polymer solution is sprayed in the form of a mist, an air spray, an air brush or the like can be used.

After the water-soluble neutral polymer is adsorbed on the aluminum oxide by the method illustrated above, a centrifugation operation may be carried out to remove an excess polymer solution, or the aluminum oxide is directly used for collecting the cell-free DNA without the removal operation. When the polymer solution is dissolved in a solvent, after the water-soluble neutral polymer is adsorbed on the aluminum oxide, the solvent may be removed by drying or the aluminum oxide may be used directly for collecting the cell-free DNA without drying.

The obtained support of the present invention may be stored and then used, or may be prepared at time of use.

When the obtained polymer is solid, the polymer solution can be prepared by dissolving the polymer in water or an organic solvent and, when the obtained polymer is a solution, the polymer solution can be prepared by diluting the solution. When it is hard to dissolve the polymer or mix the polymer due to the high viscosity of the solution, a heating treatment or sonication may be performed. As the organic solvent, a solvent, such as ethanol, acetonitrile, methanol, propanol, tert-butanol, DMF, DMSO, acetone, ethylene glycol and glycerol, which is miscible with water is preferably used. When the polymer is poorly soluble in water, an organic solvent described above may be added.

A support produced by binding covalently the aluminum oxide and the water-soluble neutral polymer by, for example, a linker molecule is not the support of the present invention. Specific examples of linker molecules include silane coupling agents.

Thereinafter, the present invention will be described for each step.

The step a) is a step of mixing an aluminum oxide support comprising a water-soluble neutral polymer adsorbed on a surface thereof, wherein the support is produced by adsorbing a water-soluble neutral polymer onto the surface of aluminum oxide, which is prepared by the above-mentioned method (herein described as the support of the present invention) with a body fluid sample to allow cell-free DNA to be adsorbed onto the support of the present invention, wherein the polymer is polyethylene glycol, polyvinyl alcohol, polyvinylpyrrolidone, poly(2-ethyl-2-oxazoline) or hydroxypropylmethylcellulose. The method of mixing the support of the present invention with the body fluid sample is not particularly limited, but may be carried out, for example, by pipetting or mixing by inversion, or using an instrument such as a mixer or vortex. The mixing time is not particularly limited, but may be about 5 minutes, or may be a longer time. The support of the present invention may be packed in a column and the body fluid sample may be allowed to pass through the column. Furthermore, in mixing the support of the present invention with the body fluid sample, a protein denaturant may be added. The protein denaturant may be added in advance into the body fluid sample as a preliminary stage for the step a).

The step b) is a step of separating the support on which cell-free DNA was adsorbed from the mixture mixed in the step a). Examples of separation methods include a method in which the mixture obtained in the step a) is centrifuged, the support on which cell-free DNA is adsorbed is allowed to precipitate, and then the supernatant is removed. Since the specific gravity of the support on which cell-free DNA is adsorbed is greater than that of water, the precipitation can be done easily by the centrifugation operation. Conditions for centrifugation may be a treatment at 6000 G for 1 minute, and more preferably a treatment at 10000 G for 1 minute. Other separation methods include a method in which an ultrafiltration membrane is used. The mixture obtained in the step a) is allowed to pass through an ultrafiltration membrane having a smaller pore diameter than the particle size of the support on which cell-free DNA is adsorbed, and thus the support is separated. Such an ultrafiltration membrane is available as a kit, and a centrifugal filter kit as typified by Ultrafree (registered trademark) manufactured by Merck Ltd., or Nanosep (registered trademark) manufactured by Pall Corporation can be obtained for use.

After the operation of the step b), the treatment as described below may be carried out if necessary. This is because that a biological sample-derived material other than the cell-free DNA of interest may be adsorbed on the surface of the support of the present invention. For example, washing or degradation treatment can be performed in order to isolate the cell-free DNA in higher purity. Specifically, various treatments, can be performed, such as washing with water to remove non-specifically adsorbed compounds; washing with a surfactant to remove non-specifically adsorbed proteins; washing with a surfactant-containing solution to remove ions and low-molecular compounds; washing with an organic solvent to remove non-specifically adsorbed hydrophobic compounds; adding a protease to degrade non-specifically adsorbed proteins; adding a ribonuclease to isolate only DNA, and adding DNA nuclease to isolate only RNA.

The step c) is a step of collecting the cell-free DNA by adding an eluent to the support on which cell-free DNA is adsorbed which support was separated in the step b). It is a step in which an eluent is added to the support on which cell-free DNA is adsorbed, thereby allowing the adsorbed cell-free DNA to be eluted into the eluent and thus the cell-free DNA is collected.

In the step c), examples of the methods of separating the support of the present invention from the solution which is used for eluting the cell-free DNA include a method of, in the step c), centrifuging the mixture obtained by adding the eluent to the support on which the cell-free DNA is adsorbed to precipitate the support of the present invention, and obtaining the supernatant into which the cell-free DNA is eluted. Since the relative gravity of the support of the present invention is greater than that of water, the support can be easily allowed to precipitate by the centrifugation operation. Conditions for centrifugation may be a treatment at 6000 G for 1 minute, and preferably a treatment at 10000 G for 1 minute.

Other separation methods include a method in which an ultrafiltration membrane is used. The mixture obtained in the step c) is allowed to pass through an ultrafiltration membrane with a smaller pore size than the particle size of the support of the present invention, which results in separating the support of the present invention. Such an ultrafiltration membrane is available as a kit, and a centrifugal filter kit as typified by Ultrafree (registered trademark) manufactured by Merck Ltd., or Nanosep (registered trademark) manufactured by Pall Corporation can be obtained for use.

The cell-free DNA thus collected can be chemically modified as necessary. Examples of chemical modifications include fluorescent dye modification, quencher modification, biotin modification, amination, carboxylation, maleimidation, succinimidation, phosphorylation and dephosphorylation, to an end of the cell-free DNA, and the other examples include intercalator staining. These modifications may be introduced by chemical reaction, or may be introduced by enzyme reaction. These modification group is introduced prior to the above-mentioned quantification, and the modification group introduced via chemical modification is quantified instead of quantifying collected cell-free DNA itself, whereby the cell-free DNA can be indirectly quantified.

Using the cell-free DNA collected by the method of collecting cell-free DNA according to the present invention, a genetic mutation specific to cancer can be detected, or a cancer can be detected. Thereinafter, these detection will be described.

A genetic mutation specific to cancer can be detected by collecting cell-free DNA from a body fluid sample of a subject using the method of collecting cell-free DNA according to the present invention and then analyzing the gene sequence of the cell-free DNA. The cancer to be detected and the gene sequence in which a mutation is identified in the cancer can be selected from cancers and sequences reported in a database (http://cancer.sanger.ac.uk/cosmic) such as "Catalogue Of Somatic Mutations In Cancer." Specifically, the following genes can be included as examples.

Examples of genes in which a mutation are observed in case of lung cancer include genes such as AKT1, ALK, APC, ATM, BAI3, BAP1, BRAF, CDKN2A, EGFR, EPHA5, ERBB2, ERBB4, FBXW7, FGFR1, FGFR2, GRM8, KDR, KEAP1, KIT, KMT2D, KRAS, LRP1B, MDM2, MET, MLH1, MUC16, MYC, NF1, NFE2L2, NOTCH1, PDGFRA, PIK3CA, PIK3CG, PKHD1, PTEN, RARB, RB1, RET, ROS1, RUNX1T1, SMAD4, SMARCA4, SOX2, STK11, TP53.

Examples of genes in which a mutation are observed in case of breast cancer include genes such as ACVR1B, AKT1, ATM, BAP1, BRCA1, BRCA2, CBFB, CDH1, CDKN2A, EGFR, EP300, ERBB2, ERBB3, ESR1, EXOC2, EXT2, FBXO32, FGFR1, FGFR2, GATA3, IRAK4, ITCH, KMT2C, MAP2K4, MAP3K1, MDM2, MUC16, MYC, NCOR1, NEK2, PBRM1, PCGF2, PIK3CA, PIK3R1, PPM1L, PTEN, PTGFR, RB1, RET, SEPT9, TP53, TRAF5, WEE1, ZBED4.

Examples of genes in which a mutation are observed in case of colon cancer include genes such as ACVR1B, AKT1, APC, ATM, ATP6V0D2, BAX, BRAF, CASP8, CDC27, CTNNB1, DCC, DMD, EP300, ERBB2, FBXW7, FZD3, GPC6, KRAS, MAP2K4, MAP7, MIER3, MLH1, MSH2, MSH3, MSH6, MYO1B, NRAS, PIK3CA, PIK3R1, PTPN12, SLC9A9, SMAD2, SMAD4, TCERG1, TCF7L2, TGFBR2, TP53, WBSCR17.

Examples of genes in which a mutation are observed in case of myeloproliferative neoplasms include genes such as ABL1, ASXL1, ATRX, BCOR, BCORL1, CBL, CBLB, DAXX, DNMT3A, EED, ETV6, EZH2, FLT3, GATA1, GNAS, IDH1, IDH2, IKZF1, JAK1, JAK2, JAK3, KAT6A, KIT, KMT2A, KRAS, MPL, NF1, NPM1, NRAS, PHF6, PRPF40B, PTPN11, RAD21, RB1, RUNX1, SETBP1, SF1, SF3A1, SF3B1, SH2B3, SMC1A, SMC3, STAG2, SUZ12, TET2, TP53, U2AF1, U2AF2, WT1, ZRSR2.

Examples of genes in which a mutation are observed in case of liver cancer include genes such as ALB, AMPH, APC, ARID1A, ARID2, ATM, AXIN1, BAZ2B, BRAF, CCDC178, CDKN2A, CSMD3, CTNNB1, DSE, ELMO1, ERBB2, ERRFI1, GXYLT1, HNF1A, IGF2R, IGSF10, KEAP1, KRAS, MET, OTOP1, PIK3CA, SAMD9L, TP53, UBR3, USP25, WWP1, ZIC3, ZNF226.

Examples of genes in which a mutation are observed in case of ovarian cancer include genes such as AKT1, ARID1A, BRAF, BRCA1, BRCA2, CBLC, CCNE1, CDK12, CDKN2A, CSMD3, CTNNB1, CUBN, EGFR, ERBB2, FAT3, GABRA6, KIT, KRAS, KREMEN1, MAS1L, MLH1, MSH2, NF1, NRAS, PDGFRA, PIK3CA, PIK3R1, PPP2R1A, PTEN, RB1, TP53, USP16.

Examples of genes in which a mutation are observed in case of prostate cancer include genes such as AKAP9, APC, AR, CDK12, CDKN1B, CDKN2A, GLI1, IKZF4, KDM4B, KLF6, KMT2D, MED12, MYC, NCOA2, NIPA2, NKX3-1, NRCAM, OR5L1, PDZRN3, PIK3CA, PTEN, RB1, SCN11A, SPOP, SYNE3, TBX20, TFG, THSD7B, TP53, ZFHX3, ZNF473, ZNF595.

Examples of genes in which a mutation are observed in case of gastric cancer include genes such as APC, ATP4A, BAI3, BRCA2, CCNE1, CDH1, CTNNB1, DCC, ERBB2, FBXW7, FGFR2, GPR78, LPAR2, LRP1B, LRRK2, MET, MYC, NOTCH1, PIK3CA, PRKDC, RET, S1PR2, SPEG, SSTR1, STK11, TP53, TRIO, TRRAP, WNK2.

As a method of detecting these genetic mutation, known methods of detecting nucleic acids can be used. Specifically, a genetic mutation of interest is selected and detected using PCR, real-time PCR, digital PCR, and an instrument for detecting nucleic acids such as a sequencer and microarray, using a primer and probe which can detect specifically to a genetic mutation of a gene of interest. With respect to presence or absence of the genetic mutation, if significantly higher signal than a measured value of a negative control in each detection method is measured, then it can be determined that a genetic mutation is present. More preferably, a specimen which has been identified previously to have no genetic mutation and a specimen of a subject is measured for genetic mutations, and if there is a statistically significant difference between a measured value obtained from the specimen which has been identified previously to have no genetic mutation and a measured value obtained from the specimen of the subject and also if the measured value obtained from the specimen of the subject is higher, then it can be determined that a mutation of gene of interest is present and has been detected.

Also, a cancer can be detected by collecting cell-free DNA from body fluid samples of a subject and a specimen without cancer using the method of collecting cell-free DNA according to the present invention, and comparing the amount of cell-free DNA from the subject with the amount of cell-free DNA from the specimen without cancer. Specifically, the amount of the cell-free DNA from the subject is compared with the amount of the cell-free DNA from the specimen without cancer and, as the result, if the amount of the cell-free DNA from the subject is more than the amount of the cell-free DNA from the specimen without cancer, then it can be decided that a cancer is detected from the specimen from the subject. Desirably, when there is a statistically significant difference between the amount of the cell-free DNA from the subject and the amount of the cell-free DNA from the specimen without cancer, if the amount of the cell-free DNA from the subject is more, then it can be decided that a cancer is detected.

A specimen without cancer used in the present invention is a specimen from a human who has no previous history that malignant tumor was clinically diagnosed or confirmed by pathologic examination. The physical condition of a specimen without cancer is preferably equal to or approximated as a subject. A physical condition corresponds to, for example, race or the like.

As the kind of a body fluid sample from a subject and a specimen without cancer, the same kinds of the body fluid samples are preferably used. Specifically, when a specimen from a subject is blood plasma, a specimen without cancer is preferably also blood plasma. Similarly, when a specimen from a subject is blood serum, a specimen without cancer is preferably also blood serum.

In addition, a single or a plurality of specimens without cancer may be used. When a plurality of specimens without cancer are used, an averaged value, an averaged value excluding outliers, median value, or the like can be used as the amount of the cell-free DNA.

For the method of quantifying the amount of the collected cell-free DNA, the method mentioned above as a method of quantifying the amount of a nucleic acid can be used.

In this instance, the length of cell-free DNA to be quantified is not particularly limited, but cell-free DNA having the length of 100 bp or more is preferred, and cell-free DNA having the length of 300 bp or more is more preferred. In addition, a gene sequence to be quantified is not particularly limited as long as the sequence is contained in cell-free DNA, but, it may be, for example, a housekeeping gene such as ACTB or GAPDH or may be a cancer characteristic gene. As the amounts of the cell-free DNA of the specimen without cancer and the subject, the measured values measured by the same method are preferably used.

As used herein, "statistically significant difference" means that, for example" it can be decided whether a measured value of a subject reaches a baseline when the lower limit of 95% confidence interval of a control group is set as the baseline. When a critical rate (significance level) of the obtained value is small, specifically, such a case includes a case that p < 0.05, p < 0.01 or p < 0.001. Here, the critical rate "p" or "p value" indicates, in a statistical test, a probability that an assumption is accidentally true among the distribution assumed by the statistics. Therefore, as "p" or "p value" is smaller, it means that the assumption is closer to true. As the statistical methods for statistical procedures, known test methods in which the presence or absence of significance can be determined may be appropriately used, and the statistical methods for statistical procedures are not particularly limited. For example, Student's t-test, or multiple comparison test can be used.

### [EXAMPLES]

The present invention will be more specifically described by way of the following Examples.

### <Materials and Methods>

Polyethylene glycol was purchased from Merck Ltd., and basic gamma aluminum oxide (N613N) was purchased from JGC Catalysts and Chemicals Ltd. The aqueous polymer solutions used in Examples were obtained by dissolving polymers in water to be in each concentration. In Examples, unless otherwise specified, basic gamma aluminum oxide was used. The aluminum oxide was used in the experiments directly after the purchase without a sieving process or the like.

As a denaturant, QIAGEN, Proteinase K and RLT were purchased from QIAGEN K.K., and urea was purchased from Wako Pure Chemical Industries, Ltd.

Also, 20 bp DNA ladder which was used for electrophoresis was purchased from TAKARA BIO INC. and acrylamide gel NOVEX-TBE-Gells 8% 15 well and DNA stain SYBR-Gold Nucleic Acid Gel Stain was purchased from Thermo Fisher Scientific Inc. For real-time PCR, SYBR (registered trademark) Premix Ex Taq II from TAKARA BIO INC. was used. For detecting cell-free DNA, SEQ NO: 1 and SEQ NO:2 were used as primers for amplifying the length of 93 bp of the gene sequence of beta-actin (ACTIN 93). For detecting a cancer by an amount of cell-free DNA, SEQ NO: 1 and SEQ NO:3 were used as primers for amplifying the length of 306 bp of the gene sequence of beta-actin. A primer was designed based on the description of PrimePCR Assays, Panels, and Controls Instruction Manual (Bio-Rad Laboratories, Inc.), purchased from Eurofins Genomics K.K., and used directly as purchased without any particular purification. Other reagents were purchased from Wako Pure Chemical Industries, Ltd., Tokyo Chemical Industry Co., Ltd., and Sigma-Aldrich Japan, and used directly as purchased without any particular purification.

CUTE MIXER CM-1000 from TOKYO RIKAKIKAI CO, LTD. was used for vortexing, and ELS-Z from Otsuka Electronics Co., Ltd. was used for measuring a zeta potential. Mini Gel Slab Electrophoretic Device (AS ONE Corporation) was used for electrophoresis. PCR Thermal Cycler SP TP-400 from TAKARA BIO INC. was used as a thermal cycler, and CFX 96-Real Time System from Bio-Rad Laboratories, Inc. was used for real-time PCR. For a sieve, MVS-1 from AS ONE Corporation was used. Stained gel was analyzed using Typhoon FLA 9500 from GE Healthcare Japan Corporation. ImageQuant TL (registered trademark) from Molecular Dynamics was used for image-analyzing for gels.

In quantifying cell-free DNA in real-time PCR, as the result of real-time PCR, it was decided that cell-free DNA could be collected when the number of amplification cycles (Cq value) is 35 or less. When the number of amplification cycles (Cq value) is 40 or more, since it is decided that cell-free DNA is below the lower limit of measurement, it is represented as N.D.

For body fluid samples, mixed blood plasma from a plurality of persons, purchased from Tennessee Blood Services, and blood plasma from females and males who do not suffer from any cancer, purchased from Cureline, Inc. were used as a specimen without cancer. Blood plasma from patients with cancers, obtained from Cureline, Inc. were used as a specimen suffering from a cancer.

### <Comparative Example 1> Collecting cell-free DNA by using an aluminum oxide support without polymer coating

Basic gamma aluminum oxide (N613N: JGC Catalysts and Chemicals Ltd.) having a similar composition as the aluminum oxide A described in Patent Document 4 (Example 4, Table 2) was used to examine whether cell-free DNA could be collected efficiently or not. Mixed blood plasma from a plurality of persons, purchased from Tennessee Blood Services, was used as a body fluid sample. As an eluent for eluting cell-free DNA adsorbed on an aluminum oxide, Patent Documents 4 and 5 describes that a phosphate buffer solution or a Tris-EDTA buffer solution can be used and Patent Document 6 describes that a phosphoric acid solution inhibits binding a nucleic acid to an aluminum oxide, and therefore a phosphate buffer solution (0.5 M, pH 7) was used as an eluent to perform the following experiment.

Each 0.5 mg of gamma aluminum oxide was weighed into a 1.5-ml tube. To it, 400 µL was added, and the mixture was vortexed and centrifuged by a centrifuge (10000 G, 1 minute), and the supernatant was then removed. This procedure was repeated again, and dust and the like on the surface of the bead were then removed with ethanol.

To gamma aluminum oxide after finishing cleaning, 300 µL of plasma was added and vortexed for 15 minutes. The mixture was centrifuged by a centrifuge (10000 G, 1 minute) and the supernatant was then removed to remove the plasma. Subsequently, 400 µL of sterile distilled water was added, vortexed and centrifuged by a centrifuge (10000 G, 1 minute), and the supernatant was then removed. This procedure was repeated again, and remaining plasma in the tube was then completely removed with sterile distilled water. After the sterile distilled water in the tube was removed as possible, 50 µL of a phosphate buffer solution (0.5 M, pH 7) was added as an eluent and the mixture was immediately vortexed for 15 minutes. The mixture was centrifuged by a centrifuge (10000 G, 1 minute), and the supernatant containing a cell-free DNA was then collected.

The length of 93 bp of the gene sequence of beta-actin (ACTIN 93) was amplified by real-time PCR in order to determine the amount of the collected cell-free DNA. SEQ NO: 1 and SEQ NO: 2 were used as an amplification primer. The real-time PCR was performed as the following procedure.

First, 12.5 µL of SYBR Premix Ex Taq, 1.0 µL of the primer prepared to be 0.5 µM, 8.5 µL of sterile distilled water, and 2 µL of the portion of a solution, which was prepared in such a way that the sample containing the cell-free DNA collected by the above-mentioned method was diluted 10-fold with sterile distilled water, were mixed in a 1.5-ml tube on ice (Total 25 µL). All amount of 25 µL was added to a plate for real-time PCR, and the plate was capped with a plate sheet and installed to the instrument. For the measurement condition for real-time PCR, double-strand DNA was divided into two single-strand DNAs at 95°C for 30 seconds and elongation at 56°C for 1 minute was subsequently carried out 40 cycles to obtain a number of amplification cycles from the Amplification Curve. After the PCR reaction, the temperature of the reaction solution was allowed to gradually increase from 60°C to 95°C and melting curve analysis was then carried out to confirm, from the obtained MeltCurve, that any primer dimers were not produced.

The results are shown in Table 1. The number of amplification cycles (Cq value) of the real-time PCR was 40 or more which was below the detectable limit.

These results showed that, when gamma aluminum oxide on which the polymer was not adsorbed, the cell-free DNA could not be collected. Therefore, it was shown that using the method described in Patent Documents 4 and 5, no cell-free DNA could be collected.

**[Table 1]**

| Support | Coating polymer | ACTIN 93 (Cq value) | |
|---|---|---|---|
| Alumina | None | N.D. | Comparative Example 1 |

### <Comparative Example 2> Collection of cell-free DNA using an aluminum oxide support on which a water-soluble polymer other than a water-soluble neutral polymer is adsorbed

Each 0.5 mg of gamma aluminum oxide was weighed into a 1.5-ml tube. As a polymer solution, polyacrylic acid (PAcA, 5.1 kD, 10 wt%), dextran sulfate (DS, 4 kD, 10 wt%), polyvinyl sulfonic acid (PVSA, 10 wt%), polyallylamine (PAA, 17 kD, 10 wt%), and poly-L-lysine (PLL, 150 kD, 1 wt%) in an amount of 50 µl each were individually added to the respective these tubes, and respective mixtures were stirred for 10 minutes by a mixer. The mixtures were centrifuged by a centrifuge (10000 G, 1 minute), and the supernatant was removed to obtain gamma aluminum oxide with each polymer adsorbed on the surface thereof.

After mixing in advance 450 µL of RLT (QIAGEN K.K., Buffer RLT) containing guanidine hydrochloride salt as a protein denaturant with 300 µL of blood plasma used in Comparative Example 1 for an aluminum oxide support on which a water-soluble polymer other than a water-soluble neutral polymer is adsorbed, the mixture was added to the gamma aluminum oxide support comprising the polymer adsorbed on the surface thereof. The other operations were carried out in the same way as in Comparative Example 1. Also, detecting the collected cell-free DNA was confirmed in the same way as in Comparative Example 1.

The results are shown in Table 2. These results show that, in case that any supports were used, the amount of collected cell-free DNA was below the lower limit of detection of real-time PCR. These results show that, when gamma aluminum oxide on which a water-soluble polymer other than a water-soluble neutral polymer is adsorbed, the number of amplification cycles (Cq value) is 35 or more or below the detectable limit (N.D) and no cell-free DNA could be therefore collected.

**[Table 2]**

| Support | Coating polymer | Zeta potential (mV) | ACTIN93 (Cq value) | |
|---|---|---|---|---|
| Alumina | PAcA | -37 | 37.6 | Comparative Example 2 |
| | DS | -18 | N.D. | Comparative Example 2 |
| | PVSA | -17 | N.D. | Comparative Example 2 |
| | PAA | 11 | N.D. | Comparative Example 2 |
| | PLL | 14 | N.D. | Comparative Example 2 |

### <Comparative Example 3> Collection of cell-free DNA by using a silica support

A kit for collecting a cell-free DNA using a silica support (Thermo Fisher Scientific Inc., MagMax cell-Free DNA Isolation kit) was used to examine whether a cell-free DNA could be collected efficiently or not.

As a protein denaturant, Proteinase K according to the protocol of the kit or RLT (QIAGEN K.K., Buffer RLT) containing guanidine hydrochloride salt was used. As a body fluid sample, blood plasma as in Comparative Example 1 was used. When RLT was used as a protein denaturant, 300 µL of blood plasma and 450 µL of RLT used in Comparative Example 1 were mixed in advance, and the mixture was added to a silica support. The other collection procedure was carried out according to the protocol of the kit.

Also, detecting the collected cell-free DNA was confirmed in the same way as in Comparative Example 1.

The results are shown in Table 3. These results shows that Cq value is 35 or more when the silica support was used.

Furthermore, the result from gel electrophoresis of the collected cell-free DNA is shown in Fig. 1. The result of the gel electrophoresis shows that no band for the collected cell-free DNA was identified.

**[Table 3]**

| Support | Denaturant | ACTIN93 (Cq value) | |
|---|---|---|---|
| Silica | Proteinase K | 36.7 | Comparative Example 3 |
| | RLT | 36.5 | Comparative Example 3 |
| PEG Coating alumina | Proteinase K | 33.8 | Example 1 |
| | RLT | 27.7 | Example 2 |
| | Urea | 32.5 | Example 3 |
| | None | 33.8 | Example 4 |

### <Example 1> Collection of cell-free DNA by using gamma aluminum oxide support comprising water-soluble neutral polymers, PEG, adsorbed on the surface thereof (protein denaturant: Proteinase K)

Each 0.5 mg of gamma aluminum oxide was weighed into a 1.5-ml tube. To it, 400 µL of ethanol was added, and the mixture was vortexed and centrifuged by a centrifuge (10000 G, 1 minute), and the supernatant was then removed. This procedure was repeated again, and dust and the like on the surface of the bead were then removed with ethanol. As a polymer aqueous solution, 50 µL of polyethylene glycol (PEG, 10 kDa, 10 wt%), which is a water-soluble neutral polymer, was added to the respective tubes, and stirred for 10 minutes by a mixer. The mixture was centrifuged by a centrifuge (10000 G, 1 min), and the supernatant was removed to obtain gamma aluminum oxide support with each polymer adsorbed on the surface thereof.

Subsequently, 300 µL of the blood plasma used in Comparative Example 1, which blood plasma was denatured at 60°C for 20 minutes. with Proteinase K as a protein denaturant in order to be the same blood plasma condition as the kit protocol in Comparative Example 3, was added to the solution containing the gamma aluminum oxide support comprising the polymer on the surface thereof, and vortexed for 15 minutes. The subsequent operations were carried out in the same way as in Comparative Example 1. The amount of the collected cell-free DNA was determined in the same way as in Comparative Example 1. The results are shown in Table 3.

### <Example 2> Collection of cell-free DNA by using a gamma aluminum oxide support comprising a water-soluble neutral polymer, PEG, adsorbed on the surface thereof (protein denaturant: Buffer RLT)

The denaturant for denaturing the blood plasma protein in Example 1 was changed to RLT (QIAGEN K.K., Buffer RLT) containing guanidine hydrochloride salt. As a method of the use, after mixing 300 µL of the blood plasma used in Comparative Example 1 and 450 µL of RLT in advance, the mixture was added to the gamma aluminum oxide support comprising the polymer on the surface thereof. The other operations were carried out in the same way as in Comparative Example 1. The results are shown in Table 3.

Furthermore, the result from gel electrophoresis of the collected cell-free DNA is shown in Fig. 1. From these results, in contrast to no observation of any band for the cell-free DNA collected in Comparative Example 3, the band for the cell-free DNA collected in Example 2 could be observed in the vicinity of 160 bp, and it was therefore shown that the cell-free DNA was collected in high purity by the method according to the present invention.

### <Example 3> Collection of cell-free DNA by using gamma aluminum oxide support comprising a water-soluble neutral polymer, PEG, adsorbed on the surface thereof (protein denaturant: urea)

The type of the denaturant for denaturing the blood plasma protein in Example 1 was changed to urea. As a method of the use, after mixing 300 µL of the blood plasma used in Comparative Example 1 and 450 µL of urea (final concentration 6 M) in advance, the mixture was added to the gamma aluminum oxide support comprising the polymer on the surface thereof. The other operations were carried out in the same way as in Comparative Example 1. The results are shown in Table 3.

### <Example 4> Collection of cell-free DNA by using gamma aluminum oxide support comprising a water-soluble neutral polymer, PEG, adsorbed on the surface thereof (protein denaturant: none)

Without the operation of denaturing the blood plasma protein, which operation was carried out in Example 1, the blood plasma was added to the gamma aluminum oxide support comprising the polymer on the surface thereof. The operations other than that of adding 45 µL of distilled water to 300 µL of the blood plasma used in Comparative Example 1 were carried out in the same way as in Example 1. The results are shown in Table 3.

From the results of Examples 1, 2, 3 and 4 shown in Table 3, it was shown that Cq value is 35 or less, and the cell-free DNA could be collected from 300 µL of blood plasma regardless of the presence or absence, and a type of a protein denaturant when a gamma aluminum oxide support comprising a water-soluble neutral polymer adsorbed on the surface thereof was used.

### <Example 5> Collection of cell-free DNA by using gamma aluminum oxide support comprising water-soluble neutral polymers adsorbed on the surface thereof

As a water-soluble neutral polymer, each type of polymer with a different zeta potential as described below was prepared to be 10 wt% of an aqueous solution. PEG (polyethylene glycol), PVA (polyvinyl alcohol), PEOz (poly(2-ethyl-2-oxazoline), HPMC (hydroxypropylmethylcellulose), PVP (polyvinylpyrrolidone). Each polymer solution thus prepared and a gamma aluminum oxide support were mixed to make a gamma aluminum oxide support comprising each polymer adsorbed on the surface thereof. The other operations were carried out in the same way as in Example 1.

The results are shown in Table 4. Because the number of amplification cycles (Cq value) of the real-time PCR was 35 or less, it was shown that the cell-free DNA could be efficiently collected from a small amount, 300 µL, of blood plasma when a gamma aluminum oxide support comprising a water-soluble neutral polymer which had a zeta potential in the range of ± 10 mV adsorbed on the surface thereof was used.

**[Table 4]**

| Support | Coating polymer | Zeta potential (mV) | ACTIN93 (Cq value) | |
|---|---|---|---|---|
| Alumina | PVA | -4 | 34.7 | Example5 |
| | PEOz | -2.7 | 33.8 | Example5 |
| | PEG | -1.2 | 32.9 | Example5 |
| | HPMC | -0.89 | 32 | Example5 |
| | PVP | 1.1 | 32.4 | Example5 |

### <Comparative Example 4> Collection of cell-free DNA by using a silica support

Except that the blood plasma from females and males who do not suffer from any cancer, purchased from Cureline, Inc., and the blood plasma from a patient (female) suffering from a breast cancer, purchased from Cureline, Inc., were used as a body fluid sample, collecting cell-free DNA was carried out in the same way as in Comparative Example 3, and the DNA concentration was measured using Quantus Fluorometer (registered trademark). The results are shown in Table 5.

From these results, it was shown that any specimens were 0.005 ng/µL which is below the limit of measurement and no cell-free DNAs could be therefore collected from 300 µL of the blood plasma from the specimen without cancer and the blood plasma from the patient with a cancer when the silica support was used.

### <Example 6> Collection of cell-free DNA by using a gamma aluminum oxide support comprising a water-soluble neutral polymer, PEG, adsorbed on the surface thereof (protein denaturant: urea)

Except that the support used in Example 1 was used, the same operations as in Comparative Example 4 was carried out to collect cell-free DNA from the same specimen as in Comparative Example 4. The concentration of the collected cell-free DNA was confirmed in the same way as in Comparative Example 4. The results are shown in Table 5.

As the result, 0.0149 pg/µL of cell-free DNA was obtained from the blood plasma of a male without cancer, 0.0077 pg/µL was obtained from a female without cancer, and 0.0323 pg/µL was obtained from a patient suffering from a breast cancer.

**[Table 5]**

| | Amount of nucleic acid (ng/µL) | |
|---|---|---|
| Support | Comparative Example 4 Silica | Example 6 PEG coating Alumina |
| Male without cancer | N.D. | 0.0149 |
| Female without cancer | N.D. | 0.0077 |
| Breast cancer (female) | N.D. | 0.0323 |

| | | |
|---|---|---|
| ^{∗}N.D. (less than 0.005 ng/uL) | | |

### <Comparative Example 5> Detection of a genetic mutation in cell-free DNA from a patient suffering from a cancer using a silica support

Using a kit for collecting cell-free DNA using a silica support (Thermo Fisher Scientific Inc., MagMax cell-Free DNA Isolation kit), cell free DNA was collected from 300 µL of a body fluid sample and a cancer-specific genetic mutation was then detected. The blood plasma from females and males who do not suffer from cancer, purchased from Cureline, Inc., and the blood plasma from a patient (male) suffering from a lung cancer, purchased from Cureline, Inc., were used as a body fluid sample. A method of collecting cell-free DNA was carried out in the same way as the method in which RLT was used as a denaturant in Comparative Example 3. One twenty-fifth volume of the obtained eluent was prepared to be 20 µL, and EGFR exon 19 deletion mutation which is specific to a lung cancer was detected by digital PCR. The results are shown in Table 6. As the result, though no genetic mutation was detected in specimens from a male and female without cancer, EGFR exon 19 deletion mutation was detected in a frequency of 49.45% from the cell-free DNA of the patient with a lung cancer.

### <Example 7> Detection of a genetic mutation in cell-free DNA collected by using a gamma aluminum oxide support comprising PEG adsorbed on the surface thereof

Cell-free DNA was collected from 300 µL of the blood plasma from the male and female without cancer and the patient suffering from a lung cancer which were the same specimens as in Comparative Example 5 using the same method as the method in which RLT was used as a denaturant in Comparative Example 3, and the cancer-specific genetic mutation was detected. One twenty-fifth volume of the obtained eluent was prepared to be 20 µL, and EGFR exon 19 deletion mutation which is specific to a lung cancer was detected by digital PCR. As the result, though no genetic mutation was detected in a specimens from a male and female without cancer, EGFR exon 19 deletion mutation was detected in a frequency of 55.82% from the cell-free DNA of the patient with a lung cancer. Since this value was larger than the detection frequency of genetic mutation obtained by the method in Comparative Example 5 (49.45%) (Table 6), it was shown that a cancer-specific genetic mutation could be detected by the method according to the present invention in a higher sensitivity than by the conventional methods.

**[Table 6]**

| | Detection ratio of genetic mutation (%) | |
|---|---|---|
| | Comparative Example 5 Silica | Example 7 PEG coating alumina |
| Male without cancer | 0 | 0 |
| Female without cancer | 0 | 0 |
| Lung cancer (Male) | 49.45 | 55.82 |

### <Comparative Example 6> Detection of a cancer by the amount of cell-free DNA collected by using a silica support

Using a kit for collecting cell-free DNA using a silica support (Thermo Fisher Scientific Inc., MagMax cell-Free DNA Isolation kit), cell free DNA was collected from 300 µL of a body fluid sample and the presence or absence of a cancer was then detected using the amount of cell-free DNA measured by real-time PCR. The blood plasmas from females and males who do not suffer from cancer, obtained from Cureline, Inc., and the blood plasmas with a lung cancer (male), a breast cancer (female), and a colon cancer (male), obtained from Cureline, Inc., were used as a body fluid sample. A method of collecting cell-free DNA was carried out in the same way as the method in which RLT was used as a denaturant in Comparative Example 3. DNA was eluted into 50 µL of eluent, it was 10-fold diluted and 4 µL was dispensed from the diluted solution. A fragment of about 306 bp of gene with actin sequence which was amplified using the primers of SEQ NO: 1 and SEQ NO:3 designed to detect more easily cell-free DNA from a patient with a cancer was detected by real-time PCR. The length of base pairs to be detected was allowed to be longer than 300 bp, whereby cell-free DNA, which was hard to be released in a non-cancer cell, but was released in a cancer cell, was captured. By this method, it was tried to distinguish between a non-cancer and a cancer. As a baseline, the lower limit of 95% confidence interval of measured values of specimens from a male who does not suffer from cancer and a female who does not suffer from cancer is decided, whereby the specimen was judged to be cancer positive, that is, a cancer was detected from the specimen, when the amount of the cell-free DNA was 1.5-fold or more larger (the number of PCR cycles was less by about 0.59) and otherwise the specimen was judged to be cancer negative.

When the number of PCR cycles of the respective specimens were applied to the above-mentioned criterion, more amount of the cell-free DNA of the specimens from the patients suffering from a lung cancer and colon cancer were detected than the baseline of the specimen without cancer, and they were therefore decided to be cancer positive. On the other hand, the amount of the specimen from the patient suffering from breast cancer was overlapped the baseline of the measured values from the specimens without cancer, and it was therefore decided to be cancer negative. Therefore, because one specimen of the three cancer specimens could not be decided to have a cancer by this method, the sensitivity of this method was 66%.

### <Example 8> Detection of a cancer by the amount of cell-free DNA collected by using a gamma aluminum oxide support comprising PEG adsorbed on the surface thereof

Using the same way as the method in which RLT was used as a denaturant in Example 2, cell-free DNA was collected from 300 µL of the same body fluid sample as in Comparative Example 6, and the presence or absence of a cancer was detected using the amount of the cell-free DNA measured by real-time PCR. A method of collecting cell-free DNA was carried out in the same way as the method in which RLT was used as a denaturant in Comparative Example 3. DNA was eluted into 50 µL of eluent, it was 10-fold diluted and 4 µL was dispensed from the diluted solution. A fragment of gene with actin sequence which was amplified using the primers of SEQ NO: 1 and SEQ NO:3 designed to detect more easily cell-free DNA from a patient with a cancer was detected by real-time PCR. Decision for the presence or absence of a cancer was performed in the same way as in Comparative Example 6. As the result, more amount of the cell-free DNA of the respective blood plasma from the patients suffering from a lung cancer (male), breast cancer (female) and colon cancer (male) were detected than the baseline of the specimen without cancer, and all of them were therefore decided to be cancer positive. Therefore, because all three specimens of the three cancer specimens could be decided to have a cancer by this method, the sensitivity of this method was 100%. Since this sensitivity was higher than the sensitivity obtained by the method for Comparative Example 6 (66%) (Table 7), it was shown that the method according to the present invention allowed for detecting a cancer in higher sensitivity from cell-free DNA than the conventional methods.

**[Table 7]**

| | Comparative Example 6 (Silica) | Example 8 PEG coating alumina |
|---|---|---|
| Lung cancer (Male) | Cancer positive | Cancer positive |
| Breast cancer (Female) | Cancer negative | Cancer positive |
| Colon cancer (Male) | Cancer positive | Cancer positive |

## Claims

1. A method of collecting cell-free DNA from a body fluid sample, comprising the following steps:
step a) of mixing an aluminum oxide support comprising a water-soluble neutral polymer adsorbed on a surface thereof, wherein the support is produced by adsorbing a water-soluble neutral polymer onto the surface of aluminum oxide, with the body fluid sample to adsorb the cell-free DNA onto the support, wherein the polymer is polyethylene glycol, polyvinyl alcohol, polyvinylpyrrolidone, poly(2-ethyl-2-oxazoline) or hydroxypropylmethylcellulose
step b) separating the support on which the cell-free DNA was adsorbed from the mixture mixed in the step a), and
step c) collecting the cell-free DNA by adding an eluent to the support on which the cell-free DNA was adsorbed and which was separated in the step b).

2. The method of collecting cell-free DNA according to claim 1, wherein the body fluid sample is whole blood, blood serum, blood plasma, urine or saliva.

3. The method of collecting cell-free DNA according to claim 1 or 2, wherein the water-soluble neutral polymer is a polymer having a zeta potential of not less than -10 mV and not more than +10 mV in a solution of pH 7.

4. The method of collecting cell-free DNA according to any of claims 1 to 3, wherein the eluent is a buffer solution.

5. The method of collecting cell-free DNA according to any of claims 1 to 4, wherein the body fluid sample is treated with a protein denaturant.

6. The method of collecting cell-free DNA according to claim 5, wherein the protein denaturant is a chaotropic salt or protein-denaturing enzyme.

7. A method of detecting a genetic mutation specific to cancer by analyzing a genetic sequence of cell-free DNA, wherein the method comprises the steps of collecting the cell-free DNA from a body fluid sample of a subject using the method of collecting cell-free DNA according to any of claims 1 to 6 and detecting the genetic mutation specific to cancer from the cell-free DNA thus collected.

8. A method of detecting a cancer by comparing an amount of cell-free DNA from a subject with an amount of cell-free DNA from a specimen without cancer, wherein the method comprises the steps of collecting the cell-free DNA from the respective body fluid sample from the subject and the specimen without cancer using the method of collecting cell-free DNA according to any of claims 1 to 6, and detecting the cancer by comparing the amount of cell-free DNA from a subject with the amount of cell-free DNA from a specimen without cancer.

## Patentansprüche

1. Verfahren zum Sammeln von zellfreier DNA aus einer Körperflüssigkeitsprobe, umfassend die folgenden Schritte:
Schritt a): Mischen eines Aluminiumoxidträgers, umfassend ein wasserlösliches neutrales Polymer, das auf einer Oberfläche davon adsorbiert ist, wobei der Träger durch Adsorption eines wasserlöslichen neutralen Polymers auf der Oberfläche von Aluminiumoxid hergestellt wird, mit der Körperflüssigkeitsprobe, um die zellfreie DNA auf dem Träger zu adsorbieren, wobei das Polymer Polyethylenglykol, Polyvinylalkohol, Polyvinylpyrrolidon, Poly(2-ethyl-2-oxazolin) oder Hydroxypropylmethylcellulose ist;
Schritt b): Trennen des Trägers, auf dem die zellfreie DNA adsorbiert wurde, von dem in Schritt a) gemischten Gemisch, und
Schritt c): Sammeln der zellfreien DNA durch Zugabe eines Elutionsmittels zu dem Träger, auf dem die zellfreie DNA adsorbiert wurde und der in Schritt b) getrennt wurde.

2. Verfahren zum Sammeln von zellfreier DNA nach Anspruch 1, wobei die Körperflüssigkeitsprobe Vollblut, Blutserum, Blutplasma, Urin oder Speichel ist.

3. Verfahren zum Sammeln von zellfreier DNA nach Anspruch 1 oder 2, wobei das wasserlösliche neutrale Polymer ein Polymer ist, das ein Zetapotential von nicht weniger als -10 mV und nicht mehr als +10 mV in einer Lösung von pH 7 aufweist.

4. Verfahren zum Sammeln von zellfreier DNA nach einem der Ansprüche 1 bis 3, wobei das Elutionsmittel eine Pufferlösung ist.

5. Verfahren zum Sammeln von zellfreier DNA nach einem der Ansprüche 1 bis 4, wobei die Körperflüssigkeitsprobe mit einem Protein-Denaturierungsmittel behandelt wird.

6. Verfahren zum Sammeln von zellfreier DNA nach Anspruch 5, wobei das Protein-Denaturierungsmittel ein chaotropes Salz oder ein Protein-denaturierendes Enzym ist.

7. Verfahren zum Nachweis einer krebsspezifischen genetischen Mutation durch Analyse einer genetischen Sequenz von zellfreier DNA, wobei das Verfahren die Schritte des Sammelns der zellfreien DNA aus einer Körperflüssigkeitsprobe eines Probanden unter Verwendung des Verfahrens zum Sammeln zellfreier DNA nach einem der Ansprüche 1 bis 6 und des Nachweises der krebsspezifischen genetischen Mutation aus der so gesammelten zellfreien DNA umfasst.

8. Verfahren zum Nachweisen eines Krebses durch Vergleich einer Menge zellfreier DNA von einem Probanden mit einer Menge zellfreier DNA von einer Probe ohne Krebs, wobei das Verfahren die Schritte des Sammelns der zellfreien DNA aus der jeweiligen Körperflüssigkeitsprobe von dem Probanden und der Probe ohne Krebs unter Verwendung des Verfahrens zum Sammeln von zellfreier DNA nach einem der Ansprüche 1 bis 6 umfasst, und zum Nachweisen des Krebses durch Vergleich der Menge zellfreier DNA von einem Probanden mit der Menge zellfreier DNA von einer Probe ohne Krebs.

## Revendications

1. Procédé de collecte d'ADN acellulaire à partir d'un échantillon de fluide corporel, comprenant les étapes suivantes :
une étape a) de mélange d'un support d'oxyde d'aluminium comprenant un polymère neutre hydrosoluble adsorbé sur une surface de celui-ci, le support étant produit par adsorption d'un polymère neutre hydrosoluble sur la surface de l'oxyde d'aluminium, avec l'échantillon de fluide corporel afin d'adsorber l'ADN acellulaire sur le support, le polymère étant le polyéthylène glycol, le poly(alcool vinylique), la polyvinylpyrrolidone, la poly(2-éthyl-2-oxazoline) ou l'hydroxypropylméthylcellulose,
une étape b) de séparation du support sur lequel l'ADN acellulaire a été adsorbé à partir du mélange mélangé dans l'étape a), et
une étape c) de collecte de l'ADN acellulaire par ajout d'un éluant au support sur lequel l'ADN acellulaire a été adsorbé et qui a été séparé dans l'étape b).

2. Procédé de collecte d'ADN acellulaire selon la revendication 1, dans lequel l'échantillon de fluide corporel est du sang total, du sérum sanguin, du plasma sanguin, de l'urine ou de la salive.

3. Procédé de collecte d'ADN acellulaire selon la revendication 1 ou 2, dans lequel le polymère neutre hydrosoluble est un polymère ayant un potentiel zêta non inférieur à -10 mV et non supérieur à +10 mV dans une solution à pH 7.

4. Procédé de collecte d'ADN acellulaire selon l'une quelconque des revendications 1 à 3, dans lequel l'éluant est une solution tampon.

5. Procédé de collecte d'ADN acellulaire selon l'une quelconque des revendications 1 à 4, dans lequel l'échantillon de fluide corporel est traité avec un dénaturant de protéines.

6. Procédé de collecte d'ADN acellulaire selon la revendication 5, dans lequel le dénaturant de protéines est un sel chaotropique ou une enzyme dénaturant les protéines.

7. Procédé de détection d'une mutation génétique spécifique du cancer par analyse d'une séquence génétique d'ADN acellulaire, le procédé comprenant les étapes de collecte de l'ADN acellulaire à partir d'un échantillon de fluide corporel d'un sujet en utilisant le procédé de collecte d'ADN acellulaire selon l'une quelconque des revendications 1 à 6 et de détection de la mutation génétique spécifique du cancer à partir de l'ADN acellulaire ainsi collecté.

8. Procédé de détection d'un cancer par comparaison d'une quantité d'ADN acellulaire provenant d'un sujet à une quantité d'ADN acellulaire provenant d'un spécimen sans cancer, le procédé comprenant les étapes de collecte de l'ADN acellulaire à partir de l'échantillon de fluide corporel respectif provenant du sujet et du spécimen sans cancer en utilisant le procédé de collecte d'ADN acellulaire selon l'une quelconque des revendications 1 à 6, et de détection du cancer en comparant la quantité d'ADN acellulaire provenant d'un sujet à la quantité d'ADN acellulaire provenant d'un spécimen sans cancer.


### SEQUENCE LISTING

<110> TORAY INDUSTRIES, INC.
<120> The method for isolating cell free DNA
<130> 17194WO01
<160> 3
<170> PatentIn version 3.5
<210> 1
   <211> 22
   <212> DNA
   <213> Homo sapiens
<400> 1
   tgagatgcgt tgttacagga ag 22
<210> 2
   <211> 19
   <212> DNA
   <213> Homo sapiens
<400> 2
   gtgtggactt gggagagga 19
<210> 3
   <211> 22
   <212> DNA
   <213> Homo sapiens
<400> 3
   caagtcagtg tacaggtaag cc 22